# EUROPEAN PATENT SPECIFICATION

(11) **EP 1 529 553 B1**
(45) Date of publication and mention of the grant of the patent: **20.12.2006**
(21) Application number: 03025415.5
(22) Date of filing: 05.11.2003
(51) Int. Cl.: A61N 5/10

(54) **Test device for testing positioning of a radioactive radiation source and method using the same**
Prüfvorrichtung zur Prüfung der Positionierung einer radioaktiven Strahlungsquelle und deren Verwendungsverfahren
Dispositif de test pour le test du positionnement d'une source radioactive de rayonnement et méthode de l'utilisant

(43) Date of publication of application: 11.05.2005
(73) Proprietor: NeoVista, Inc., Duluth, Georgia 30097 (US)
(72) Inventor: Pintaske, Rainer Dr., Siedlungsstrasse 5 - 7 09509 Pockau (DE); Rothe, Stefan, Siedlungsstrasse 5 - 7 09509 Pockau (DE); Fritz, Eberhard Dr., 38110 Braunschweig (DE)
(74) Representative: Schwabe - Sandmair - Marx

(56) References cited:
- DE-A- 19 933 284
- GB-A- 1 211 316
- US-B1- 6 458 068

## Description

The present invention relates to a test device for testing proper positioning of a radioactive radiation source for brachytherapy in an applicator tip and a corrsponding method.

### Background of the invention

Radiation therapy has been used in medical applications especially for treatment of cancers with relative success for many years. To ameliorate the undesired side effects of said irradiation, brachytherapy has been developed. With this approach small sized radioactive radiation sources are introduced into the body either for short time or permanently, to deliver radiation directly to the site to be treated and to prevent irradiation of healthy tissue as far as possible. Brachytherapy has not only been used in treatment of cancers, but has also been used in e.g. in the posttreatment of cardiac surgery such as to prevent formation of scar tissue or for treatment of macula degeneration, especially age related macula degeneration.

The radiation sources used for brachytherapy are usually minimum size devices having dimensions of 0.8 to 1.0 mm diameter and about 4.5 to 5 mm length. These are inserted either through hollow needles, which deliver the radiation source to the site of treatment. This insertion method is predominantly used on permanent implants. Alternatively, the radiation sources are applied by using a catheter or an applicator employing the well-known technique of after loading. In this technology the catheter, is first put into place to reach the desired site of treatment, and, after placing the catheter the radiation source is inserted at the proximal end thereof, then advanced to the distal end of the catheter. Prior insertion of the catheter devoid of the radiation source prevents irradiation of healthy tissue during the insertion process, as the radiation source may be advanced much more quickly through the catheter, once in position, to the site of treatment.

To monitor proper positioning of the radiation source within a patient's body current technology either uses the emitted radiation or, more preferably, monitors by x-ray the progress of a radioopaque marker included in the radiation source or seed. Corresponding methods, sources and seeds are for example disclosed in European patent applications 1 060 765, 1 060 764, and 1 084 733.

Alternatively, the radiation source may be affixed to the tip of an applicator which is then quickly advanced to the site of treatment. The corresponding applications typically are used in easily accessible areas or body cavities and especially the treatment of macula degeneration. Corresponding applicators are e.g. disclosed in the US laid open publication US2002/0115902 or US patent No. 6 443 883, both concerning of ophthalmic brachytherapy devices.

In conventional brachytherapy, especially tumor therapy typically radiation sources are used, which have a circumferrentially axial symetric radiation field to axially symetrically irradiate the entire tissue surrounding the radiation source. Differing in that regard are typically radiation source used in therapy of macula degeneration. In this case patches of the neovascularisation are to be irradiated, whereas the retina, lens, vitreous and optic nerve are highly undesirably to be irradiated.

To prevent such irradiation the above-referenced US patent No. 6 443 881 for example discloses to use a partially shielded radiation source, which substantially emits radiation in one direction only. US laid open patent publication US 2002/0115902 discloses a surgical device for localized delivery of β-radiation in surgical procedures, particularly ophthalmic procedures. The preferred surgical device includes a canula with a β-radiotherapy emitting material at the distal end of the canula. The β-radiotherapy emitting material is housed in partially shielded compartment. Shielding is achieved by a thin wall metal such as stainless steel or by a thin wall polymer, plastic or similar material. The shielding may also be designed to be retractable to provide a then circumferential radiation pathway during the exposure period.

In view of the dimensions concerned and in view of the criticality to provide the radiation source at the desired site of treatment without unnecessary irradiating surrounding tissue, it is critical for the surgeon to precisely determine location of the radiation source and especially to precisely determine its positioning within the application device e.g. on the applicator or canula tip. Especially where the radiation source does not emit a circumferentially homogeneous radiation field, and the emitted radiation is rather directed due to partial shielding of the radiation source, the applicating surgeon further needs to verify or test that indeed the radiation source is positioned such to emit its radiation in the desired direction i.e. to the site to be treated and not to surrounding tissue. This means he needs not only test positioning of the source in the applicator, but also orientation of the source therein.

Of course such testing needs be done immediately before applying the source such that tests needed be carried out upon the time of surgery meaning within a sterile environment. In addition, such testing must not again contaminate the applicator or canula, but must retain the same in sterile condition. This has not been possible in the prior art.

GB-A-1211316 relates to apparatus for accurately determining the position of a radioactive source mounted in a source carrier used for example for gammagraphic monitoring or inspection of a metal component of any shape, more particularly a cylindrical or spherical shape.

### Summary of the Invention

The above objects have been solved and the drawbacks of the prior art have been overcome by providing a test device for testing proper positioning of a radioactive radiation source for brachytherapy in an applicator tip, said device comprising
- a base device containing
   - at least two radiation detector(s),
   - at least two channel(s) for guiding radiation,
   - at least two opening(s) in the surface of the base device, each channel connecting one radiation detector with one opening in the surface of the base device, and
- an applicator holder comprising a cavity for receiving the applicator tip, including the radioactive radiation source, said applicator holder further including at least one, preferably at least two opening(s), which is/are arranged to match the opening(s) in the base device, and at least one, preferably at least two collimating channel(s), which channel(s) form(s) extensions of the channel(s) in the base device through the opening(s) in the base device, and which channel(s) connect(s) separate points of the cavity with the opening(s) in the applicator holder to guide and collimate radiation emitted in separate positions of the cavity to the respective radiation detector, one of which points is located at the distal end of the cavity.
   In the second aspect the invention further relates to a method for testing proper positioning of a radioactive radiation source for brachytherapy in an applicator tip, said method comprising the steps of:
   (i) providing a test device as defined above and comprising
      - a base device containing
         - at least two radiation detector(s),
         - at least two channel(s) for guiding radiation,
         - at least two opening(s) in the surface of the base device, each channel connecting one radiation detector with one opening in the surface of the base device, and
      - an applicator holder comprising a cavity for receiving the applicator tip, including the radioactive radiation source, said applicator holder further including at least one, preferably at least two opening(s), which is/are arranged to match the opening(s) in the base device, and at least one, preferably at least two collimating channel(s), which channel(s) form(s) extensions of the channel(s) in the base device through the opening(s) in the base device, and which channel(s) connect(s) separate points of the cavity with the opening(s) in the applicator holder to guide and collimate radiation emitted in separate positions of the cavity to the respective radiation detector, one of which points is located at the distal end of the cavity;
   (ii) inserting the applicator tip including the radiation source into the applicator holder;
   (iii) attaching the applicator holder to the device; and
   (iv) detecting emitted radiation in the radiation detectors.

Preferred embodiments of the invention are as set about in the appending claims.

### Brief Description of the Drawings

In the following figures like parts are referenced by like reference numerals.

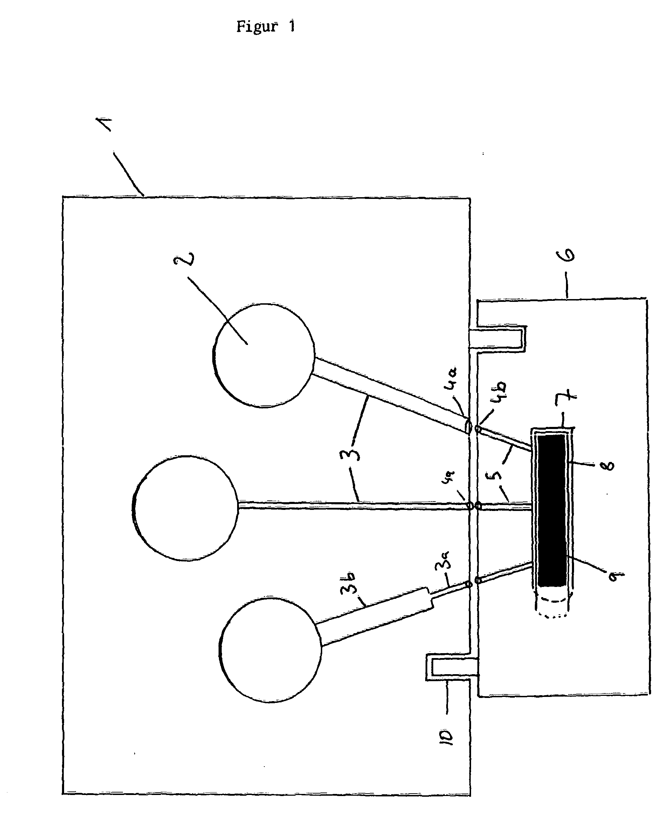

Fig. 1 is a cross-sectional view of the base device and the applicator holder of one embodiment of the invention. In this cross-section base device (1) comprises three radiation detectors (2) and three channels (3), each channel connecting one radiation detector with one opening (4a) on a surface of the base device. All three channels are straight channels and are arranged in the same plane. The channels (3) may comprise a narrow section (3a) towards the opening (4) and an enlarged or broader section (3b) towards the radiation detector (2) or may have a broader diameter throughout their entire length. The broader section (3b) may hold a collimator (not shown). Openings (4a) on the surface of the base device align and match with openings (4b) on the applicator holder (6). Applicator holder (6) comprises a cavity (7) for reviewing the applicator tip (8), including the radiation source (9) to be tested. Channels (5) in the applicator holder form extensions of channels (3) in the base device through the openings (4a/b), when aligned. Channels (5) connect openings (4b) and separate points of the cavity to guide radiation emitted in separate positions of the cavity to the respective radiation detector. Applicator holder (6) may be affixed to the base device (1) by juggle joints as shown by reference numeral 10 in Fig. 1.

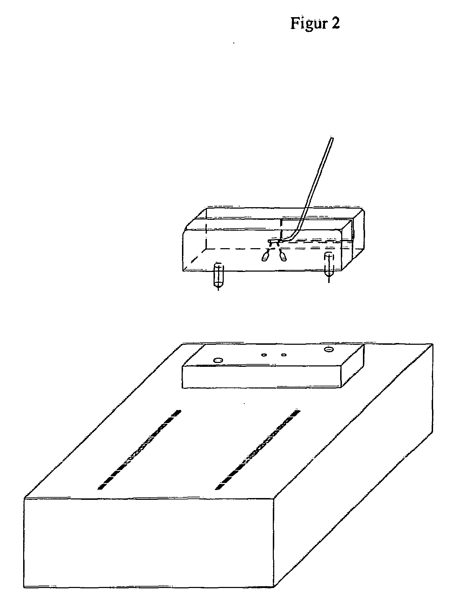

Fig. 2 shows a top view of another test device according to the present invention including base device (1), applicator holder (2), applicator (8), cavity (7), juggle joint (10) and a display in form of a bar graph chart. In this embodiment, channels (5) are shown together with respective openings (4a) in the base device to allow for guiding and detecting radiation emitted by the radiation source (not show) in applicator tip (8) by two detectors (not shown). The bar chart display shows two bars, each providing a signal proportional to the amount of radiation as detected in the respective detector. Base device (1) includes the corresponding a/d converter, electronics and is powered by mains or battery (11). As can been seen from Fig. 2, applicator holder (6) may be provided in form of two parts to simplify in insertion of the applicator tip (8) into the cavity (7). These two parts are connected by a hinge (not shown).

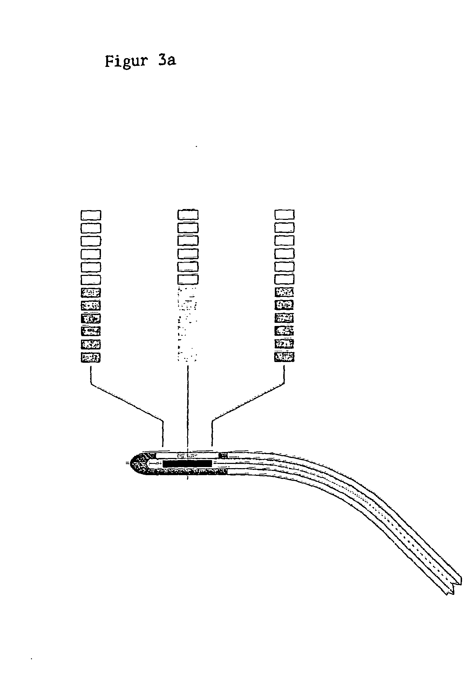

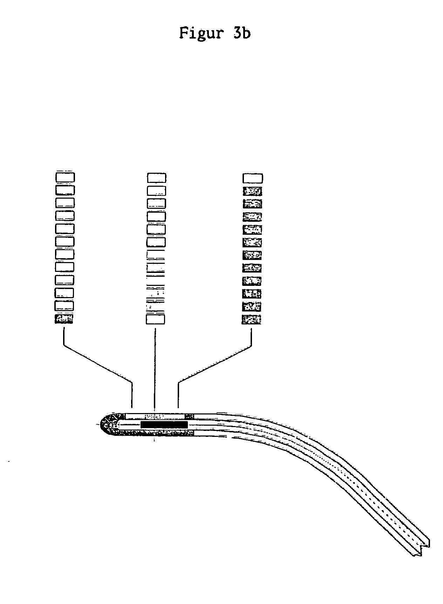

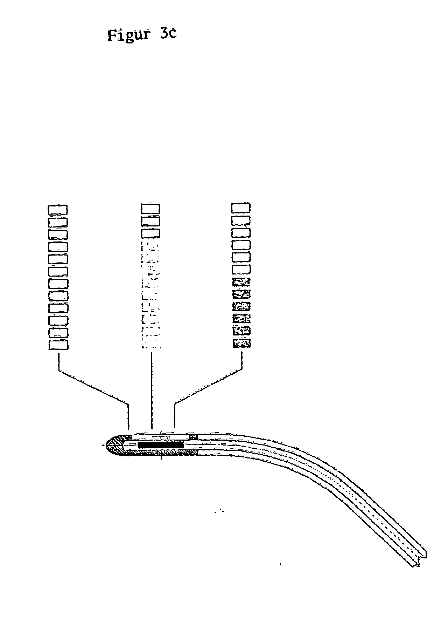

Fig. 3 shows the results obtained with differing positions of the radiation source within the applicator tip for a test device of the invention comprising three detectors, the detected radiation being shown in three corresponding bar graph displays. In Fig. 3a radiation results for proper positioning of the radiation source are provided. More in detail, in this case the same amount of radiation is received for all three channels. In case of a shielded radiation source at least the two outer channels would receive comparable amounts of radiation, whereas a smaller amount would be received in the middle detector.

Fig. 3b shows a first possibility for wrong positioning of the radiation source in the applicator tip. More in detail, here the radiation source is not located at the very end of the applicator tip, e.g. due to liquid accumulation therein. In this case the radiation detector detecting radiation emitted closely to the tip position receives little or no radiation. On the other hand the detector detecting radiation emitted at the rear end of the cavity receives a higher amount of radiation, whereas the middle position remains about unchanged.

Fig. 3c shows a comparable case for false positioning of the applicator tip, where no radiation is received by the first detector, too high radiation is observed in the middle detector (under which now the source is arranged) and about normal radiation is received for the rear end detector.

### Detailed description of the invention

The present invention relates to a test device for testing proper positioning of a radioactive radiation source for brachytherapy in an applicator tip, said device comprising
- a base device containing
   - at least two radiation detector(s),
   - at least two channel(s) for guiding radiation,
   - at least two opening(s) in the surface of the base device, each channel connecting one radiation detector with one opening in the surface of the base device, and
- an applicator holder comprising a cavity for receiving the applicator tip, including the radioactive radiation source, said applicator holder further including at least one, preferably at least two opening(s), which is/are arranged to match the opening(s) in the base device, and at least one, preferably at least two collimating channel(s), which channel(s) form(s) extensions of the channel(s) in the base device through the opening(s) in the base device, and which channel(s) connect(s) separate points of the cavity with the opening(s) in the applicator holder to guide and collimate radiation emitted in separate positions of the cavity to the respective radiation detector, one of which points is located at the distal end of the cavity.

The test device of the present invention is generally applicable to all radioactive radiation sources for use in brachytherapy regardless of the type and energy of the emitted radiation. Depending on the radiation emitted the test device will, however, be made from a suitable shielding material and will comprise an appropriate detector as is well known to the skilled worker. The only limitation as to the minimum energy required will be the necessity that direct radiation needs be received in the detector(s) of the base device.

Preferably, the test device of the present invention is used on β-radiation emitting radiation sources having a maximal β-energy of 5 MeV, preferaly 3,5 MeV for example. However, the device may also be used for soft photon emitting materials having an energy between 10 keV and 500 keV, preferably 20 and 200 keV, more preferably 20 and 100 keV for example. The radiation source to be tested typically will have a shape and size common to radiation sources known in the art. Accordingly, the radiation source will typically have a length in the order of 2 to 10 mm, preferably 4 to 5 mm. It will have a diameter between 0.1 and 1.5 mm, preferably 0.9 to 1.3 mm, the above dimensions being based on the assumption of a cylindrical shape of the source, which is most commonly used.

Applicators of use in the test device of the present invention are well-known in the art as well and include e.g. a canula, a wire or other types of applicators. Reference is made e.g. to US laid open publication US 2002/0115902, the canula of which may be used is the test device of the present invention. In general such canulas will have an elongated shape, preferably elongated cylindrical shape, with a proximal end and a distal end. The radiation source is provided at the distal end of the canula and whereas the same may have a handle extension at its proximal end for providing the surgeon with a better grip of the surgical device. The canula may in general be made of any suitable material, but is preferably made of polymeric or plastic material at its proximate end, whereas the canula or applicator to is preferably made from a thin wall metal such as stainless steel or by a thin wall polymer plastic material at the distal end/tip thereof. Its distal end or tip dimensions will be appropriate to receive and hold the radiation source to be used, without unnecessarily increasing dimensions in view of the incision size needed for insertion into the patients body. The source will typically be located at the very end or tip of the applicator.

The test device of the present invention comprises a base device and an applicator holder. These may be combined in a single unit or may form separate devices which can be aligned or attached to each other for and during measuring operations. Preferably the base device and the applicator holder form separate devices, the applicator holder forming a separable part. This embodiment allows for separate handling of the applicator holder, and especially its sterilisation before coming into contact with the applicator tip upon the measuring operation. Attachment of the applicator holder to the base device can be by any appropriate means including a juggle joint, clamp, guiding recesses etc. well known in the art. According to a preferred embodiment the attachment of the applicator holder and the base device is self-aligning or self-positioning such as assisted by gravity. To accomplish this corresponding stoppers may be provided on the base device.

The base device contains preferably at least two radiation detectors, at least two channels for guiding radiation and at least two openings in the surface of the base device. Each channel connects one radiation detector with one opening on the surface of the base device. The channels are typically built within the base device. An embodiment with one detector, one channel and one opening may in certain cases also be feasible. The channels in the base device provide for guidance of the radiation emitted directly by the source to be tested, in that only this radiation is allowed to pass. The channels in the base device may also function as collimators, though this is typically and advantageously provided for by the channels within the applicator holder.

The base device may further comprise collimators arranged in each channel or a part thereof, preferably closer to the radiation detectors. To fit those collimators, the channels may comprise a narrow section towards the opening and a broader section having a larger diameter to fit the collimator closer to the radiation detector as shown by reference numerals 3a and 3b in Fig. 1. An embodiment where the channel(s) 3 have a larger bore as shown on the right-hand side of fig. 1 may be advantageous with regard to ease of assembly and/or manufacture.

The radiation detectors for use in the base device for the present invention are conventional radiation detectors appropriate for detecting the radiation as emitted by the radiation source to be tested. The detectors can be of the type counting decay events or of the type measuring a current induced by radioactive decay. Appropriate detectors are known to the skilled worker. In case a β-radiation emitting source is to be tested, preferably beta-sensitive Geiger-Müller-counters are used.

The base device may further comprise at least one of a power supply, a pulse-shaper, a pulse-counter, means for converting the output results of the radiation detectors in electronic signals such as an a/d converter, an output means for the radiation amounts, a data processing unit and a data input unit. Preferably the base device comprises a power supply, which can be inform of a battery or a mains cable, means for converting the output results of the radiation detectors in electronic signals, if needed for the type of detector chosen, a data processing unit, and an output means for the detected radiation amount, preferably in form of a display. The display is preferably provided in form of a bar graph display, typically showing an independent bar for each radiation detector, which may optionally be supplemented with a numerical display. Other types of displays may, however, also be used. For example, the display may be a screen. Alternatively, the difference between the amounts of emitted radiation between neighbouring channels or to a predetermined or pre-establed value may be displayed either on screen or in a bar graph display.

The device of the invention preferably comprises at least two of the radiation detectors, but may comprise more than two radiation detectors, the upper number of radiation detectors to be used being determined by reasons of the practicality and costs only. Thus, in principle an indefinite number of radiation protectors could be used. The preferred number of radiation detector is in the rage of 2 to 5, most preferably 2 or 3. Each radiation detector is connected by a channel with a separate opening on the surface of the base device. Thus, the number of the channels of the base device will depend on and correspond to the number of radiation detectors provided in said device.

The base device and/or the applicator holder allow for proper shielding and guidance of the emitted radiation to the radiation detector. Thus, the applicator holder is preferably made of and the base device comprises at least e. g. as a lining for the channel(s) and, if necessary, recesses holding the detectors a material allowing (i) for appropriate guidance of the emitted radiation through the channels provided therein and (ii) for appropriate shielding of the radiation detectors from all other radiation be it emitted by the radiation source to be tested or from other sources. The holder and preferably both the base device and the holder are therefore made of a radiation shielding material of appropriate shielding capacity for the type of radiation emitted by the source to be tested. This will typically be a metallic material of an appropriated attenuation factor and manufacturing properties such as lead, tungsten, platinum, gold, aluminium, titanium, nickel, iron and their alloys and composites. The base device may, however, also be made of a plastics material optionally including metallic linings of the channels and individual metallic shields for the radiation detectors, where needed, to allow for appropriate measuring results.

The base device also includes, if desired, the above other components preferably within the same casing. The base device may further have a coating for ease of cleaning and maintenance.

The applicator holder of the test device according to the present invention comprises a cavity for receiving and holding the applicator tip including the radioactive radiation source and optionally an inlet opening for introducing the applicator tip into the cavity.

The cavity as provided in the applicator holder will have the appropriate shape and dimensions to closely hold and spatially fix the applicator tip. The end of the cavity receiving the most distal end of the applicator is designated herein as the "tip" or "distal end" of the cavity. In accordance with this the end of the cavity close to the access or inlet opening for the applicator holder will be designated herein as the "rear end" or "proximal end" of the cavity. In between a "middle part" of the cavity will be formed.

The applicator holder further includes at least two openings arranged to match the openings of the base device, once the applicator holder is attached to and/or aligned with the base device for measuring. The cavity can at least receive the entire part of the applicator/canula including the radiation source, but may be somewhat longer to avoid sharp bending of the applicator upon insertion. Preferably the "cavity" forms the end of a tunnel through and into which the applicator tip can be inserted into the applicator holder. The holder may further comprise additional means for holding e. g. an applicator handle or body.

The applicator holder further includes preferably at least two collimating channels, which channels form extensions of the at least two channels in the base device through the openings in the base device surface (4a) and applicator holder surface (4b). The channels (5) connect separate points of the cavity with the openings (4b) in the applicator holder to collimate and guide radiation emitted in separate portions of the cavity to the respective radiation detector. In general, it is apparent that the number of openings and channels in the applicator holder will match the number of openings, channels and detectors as provided in the base device.

Channels (3) in the base device and channels (5) in the applicator holder form, once base device and applicator holder are aligned with and/or attached to each other in measuring operation, a single channel connecting, preferably in a straight line a separate point of the cavity with its radiation detector. Thereby, this detector can specifically detect radiation emitted by the radiation source below said point in the cavity wall. In a preferred embodiment, the test device of the present invention includes two or three radiation detectors and two or three channels (3, 5) connecting in straight line each of the radiation detectors with separate points of the cavity. Preferably, the points in the cavity wall or openings of the channels into the cavity are arranged such that there is only little, more preferably no overlap with regard to the area or volume of the cavity from which emitted radiation is to be received. This allows for independent measuring results of the radiation detectors applied with regard to tip, middle and rear end of the cavity and radiation source, respectively. The smaller the diameter of the channels the larger the collimation effect and hence the higher precision and/or sensitivity of the measurements. This is to be traded off against practicality in view of costs and radiation dose emitted. Channels (3) and (5) may contain collimators.

In one preferred embodiment the device comprises two radiation detectors and two channels, connecting the distal and the proximal end of cavity, respectively, with one of the radiation detectors each. In another likewise preferred embodiment the device comprises three radiation detectors and three channels connecting the distal end, the middle part and the proximal end of the cavity, respectively, with one of the radiation detector with each.

In a preferred embodiment, the longitudinal axis of the applicator tip or cavity and each of the channels are arranged in the same or approximately the same plane. This arrangement not only allows for testing proper positioning of the radiation source at the tip of the applicator, but also allows for testing orientation thereof, i.e. whether indeed radiation is emitted into the desired direction, namely towards the side of the cavity, where the channels are provided.

In case more than two channels and radiation detectors are provided, the test device of the present invention may also be used to test inhomogenities of the radiation field emitted by the source. More precisely, in case radiation sources are used which a emit a intentionally inhomogeneous radiation field even towards the side the radiation is directed to in case of partially shielded radiation sources, this can likewise be tested. For example in case of three radiation detectors and a radiation pattern intending less radiation to be emitted in the middle of the source, this can easily be tested by the device of the present invention in that, in case of proper positioning of the radiation source, the amount of radiation detected by the middle radiation detector must be lower than the amount detected in the detectors at the tip and the rear end. Corresponding radiation sources are for example disclosed in the parallel pending European patent application submitted by the first applicant of the present application on the same day.

The applicator holder according to the present invention may consist of two parts, optionally connected by a joint such as a hinge. These two parts may form the cavity and the inlet for holding and receiving the applicator or canula and its tip, respectively, in this respect resembling a mould. For ease of handling, these parts may be connected by a hinge. Alternatively corresponding joints such as a juggle joint, clamp etc. may be provided.

As discussed above, the applicator holder may be made from any appropriate material. Preferably the applicator holder is made from a material of appropriate shielding capacity for the type of radiation emitted by the source to be tested. In addition, the applicator holder is preferably made of a material allowing for sterilisation of the same, typically by applying heat. Preferably at the same holder is thus made from either a metallic material of appropriate attenuation factor such as aluminum, titanium, iron, nickel or their alloys or composites. The applicator holder may, however, also be made of a plastic material optionally including parts made of a metallic material, especially the parts forming the channels and the cavity or linings therefore, to allow for appropriate shielding of radiation. In view of precision and matching with the base device, the applicator holder is preferably made from metallic materials such as aluminium, titanium, iron and nickel and alloys and composites thereof. It may have handles made of a less heat conductive material e.g. plastics, wood or ceramics for improved handling after sterilization.

A preferred embodiment of the device according to the invention is shown in cross-section in Fig. 1. In this Fig. 1 the base device is made from aluminium. Specifically, base device comprises an aluminium block of dimensions of about 30x15x10 centimetres. This aluminium block comprises appropriate recesses for receiving and holding the radiation detectors 2. Further, within the shown plane or cross-section of the base device 1 channels 3 are provided, which connect the radiation detectors 2 with openings 4a on the surface of the base device. The channels may have broader portions closer to the radiation detectors and portions having a narrow cross-section closer to the openings 4a or may have broad diameters throughout. The base device may further have appropriate electronics and may also include a display to display the amount of radiation as detected in each of the radiation detectors. These are not show in Fig. 1. In this embodiment the applicator holder is made of the same material and comprises channels and openings matching the channels and opening in the base device, if aligned and affixed to the base device in measuring operation. The applicator holder comprises a cavity 7 for receiving and holding the applicator tip 8, including the radiation source to the tested 9. Channels 5 connect specific points of the cavity with respective openings 4a on the surface of the applicator holder. These channels 5 form extensions of the channels 3 as provided in the base device. The applicator holder and the base device are connected by way of juggle joints 10. The applicator holder may further comprise an inlet for receiving the applicator body. The applicator holder typically has smaller dimensions than the base device such as a length of 10 cm and a width of 5 cm.

In the second aspect the present invention relates to a method for testing proper positioning of a radiation source for brachytherapy in a applicator tip, said method comprising the steps of:
(i) providing a test device as disclosed above and comprising
   - a base device containing
      - at least two radiation detector(s),
      - at least two channel(s) for guiding radiation,
      - at least two opening(s) in the surface of the base device, each channel connecting one radiation detector with one opening in the surface of the base device, and
   - an applicator holder comprising a cavity for receiving the applicator tip, including the radioactive radiation source, said applicator holder further including at least one, preferably at least two opening(s), which is/are arranged to match the opening(s) in the base device, and at least one, preferably at least two channel(s), which channel(s) form(s) extensions of the channel(s) in the base device through the opening(s) in the base device, and which channel(s) connect(s) separate points of the cavity with the opening(s) in the applicator holder to guide and collimate radiation emitted in separate positions of the cavity to the respective radiation detector, one of which points is located at the distal end of the cavity;
(ii) inserting the applicator tip including the radiation source into the applicator holder;
(iii) attaching the applicator holder to the device; and
(iv) detecting emitted radiation in the radiation detectors.

This method the device as described above can be used. This is provided in a first step. Upon measuring or carrying out the method in a second step an applicator tip including the radiation source to be tested is introduced through the inlet into the cavity of the applicator holder. In the third step the applicator holder is attached to the device and the emitted radiation is detected in the radiation detectors in step (iv). The method of the invention may further comprise the step of comparing the detected radiation with a pre-determined amount or pattern or comparing the detected radiation in each detector to a radiation detected in another, preferably a neighbouring detector.

The method may further comprise the steps of displaying the detected amount of radiation and/or the result of the comparison, if applicable. Such display may preferably occur by a bar graph display, the length of each bar indicating the amount of radiation detected in its coordinate detector and accordingly the amount of radiation emitted at the position connected to that respective detector by the corresponding channel. This may be used in combination with a numerical display, if desired.

As shown in Fig. 3, depending on the result given for each detector, correct positioning of the radiation source can be detected. More in detail, as shown in Fig. 3a, if the radiation source is positioned correctly, the radiation both in tip and rear position should be comparable. In case a third detector is used for the middle position, this should have the same radiation as well, in case a homogeneously emitting radiation source is tested. In case a partially shielded radiation source is used, the detected radiation ought be lower in this position. In case of an incorrect positioning of the radiation source, the detector connected with the tip of the cavity or applicator, will receive little or no radiation at all, whereas the rear end detector will receive high amounts of radiation. Also, in case of a wrong position of the applicator tip in the applicator holder, this will result in no or little radiation to be received for the tip or distal end detector, higher radiation to be detected in the middle position, and lower or average radiation to be received at the rear or proximal position detector. A corresponding bar chart is shown in Fig. 3c. Depending on the results the skilled worker may consider the respective source appropriately located for use. Accordingly, the display may instead or additionally to the detected amount of radiation even show a fail/pass signal.

The method of the invention may further comprise a step of sterilisation the applicator holder before inserting the applicator in step (ii). Sterilising the applicator holder advantageously avoids contamination of the applicator tip before inserting the same into the patient's body. The possibility of providing the holder and the base device as separate or separable entities allows such sterilisation, which might otherwise detrimentally affect the electronic components of the measuring base device. As the applicator holder itself needs not comprise such electronic parts, sterilisation becomes possible.

Although having been described with regard to preferred embodiments thereof, the skilled worker may easily device other appropriate embodiments to practice the present invention. The present invention should therefore not be construed as to be limited to these preferred embodiments. Just in contrast its scope shall be defined by the appending claims.

## Claims

1. Test device for testing proper positioning of a radioactive radiation source for brachytherapy in an applicator tip (8), said device comprising
- a base device (1) containing
- at least two radiation detectors (2),
- at least two channels (3) for guiding radiation,
- at least two openings (4) in the surface of the base device (1), each channel (3) connecting one radiation detector (2) with one opening (4) in the surface of the base device (1), and
- an applicator holder (6) comprising a cavity (7) for receiving the applicator tip (8), the cavity (7) having a distal end receiving the most distal end of the applicator tip (8) including the radioactive radiation source (9), said applicator holder (6) further including at least two openings (4a, 4b), which are arranged to match the openings (4) in the base device (1), at least two collimating channels (5), which channels (5) form extensions of the channels (3) in the base device (1) through the openings (4) in the base device (1), and which channels (5) connect separate points of the cavity (7) with the openings in the applicator holder (6) to guide and collimate radiation emitted in separate positions of the cavity (7) to the respective radiation detector (2), one of which points is located at the distal end of the cavity (7).

2. Test device of claim 1 further comprising collimators arranged in each channel (3, 5) at least in the applicator holder.

3. Test device of one of the preceding claims wherein the base (1) device further comprises at least one of a power supply (11), a pulse-shaper, a pulse-counter, means for converting the output results of the radiation detectors in electronic signals, an output means for the detected radiation amounts, a data processing unit, a display, and a data input unit.

4. Test device of one of the preceding claims wherein the applicator holder (6) is made of a radiation shielding material of appropriate shielding capacity for the type of radiation emitted by the source (9) to be tested.

5. Test device of one of the preceding claims including two or three radiation detectors (2) and two or three channels (3), connecting in straight line each of the radiation detectors (2) with separate points of the cavity (7).

6. Test device of claim 5 wherein the device comprises two radiation detectors (2) and two channels (3) connecting the distal and the proximal end of the cavity (7), respectively, with one of the radiation detectors (2) each.

7. Test device of claim 5 wherein the device comprises three radiation detectors (2) and three channels (3) connecting the distal end, the middle part and the proximal end of the cavity (7), respectively, with one of the radiation detectors (2) each.

8. Test device of one of the preceding claims wherein the longitudinal axis of the applicator tip (8) and each of the channels (3, 5) are arranged in the same plane.

9. Test device of one of the preceding claims wherein the applicator holder (6) is attached to the base device (1) such that the respective openings in the base device (1) and in the applicator holder (6) are aligned with respect to each other.

10. Test device of one of the preceding claims wherein the applicator holder (6) consists of two parts optionally connected by a hinge.

11. Test device of one of the preceding claims wherein the display is a bar graph display.

12. A method for testing proper positioning of a radiation source for brachytherapy in an applicator tip (8), said method comprising the steps of:
(i) providing a test device according to one of claims 1 to 12 comprising
- a base device (1) containing
- at least two radiation detectors (2),
- at least two channels (3) for guiding radiation,
- at least two openings (4) in the surface of the base device (1), each channel (3) connecting one radiation detector (2) with one opening (4) in the surface of the base device (1), and
- an applicator holder (6) comprising a cavity (7) for receiving the applicator tip (8), the cavity (7) having a distal end receiving the most distal end of the applicator tip (8), including the radioactive radiation source (9), said applicator holder (6) further including at least two openings (4a, 4b), which are arranged to match the openings (4) in the base device (1), and at least two channels (5), which channels (5) form extensions of the channels (3) in the base device (1) through the openings (4) in the base device, and which channels (5) connect separate points of the cavity (7) with the openings (4a, 4b) in the applicator holder (6) to guide and collimate radiation emitted in separate positions of the cavity (7) to the respective radiation detector (2), one of which points is located at the distal end of the cavity (7);
(ii) inserting the applicator tip (8) including the radiation source (9) into the applicator holder (6);
(iii) attaching the applicator holder (6) to or aligning the same with the device; and
(iv) detecting emitted radiation in the radiation detectors (2).

13. The method of claim 12 further comprising the step of comparing the detected radiation with a predetermined amount or pattern or with the radiation as detected in detector, preferably a neighboring detector another.

14. The method of one of claims 12 or 13 further comprising the step of displaying the detected amount of radiation and/or the result of the comparison.

15. The method of claim 14 wherein the display occurs by bar graph display, the length of the bar indicating the amount of radiation emitted at said position.

16. The method of one of claims 12 to 15 further comprising the step of sterilizing the applicator holder (6) before inserting the applicator tip (8) in step (ii).

17. The method of one of claims 12 to 16 wherein the base device (1) comprises two channels (3) and radiation detectors (2), and radiation emitted at the distal and the proximal end of the cavity (7) is detected.

18. The method of one of claims 12 to 16 wherein the base device (1) comprises three channels (3) and radiation detectors (2), and radiation emitted at the distal end, in the middle part and at the proximal end of the cavity is (7) detected.

19. The method of claim 14, wherein the display is in form of a fail/pass signal.

## Patentansprüche

1. Testvorrichtung zum Testen der richtigen Positionierung einer radioaktiven Strahlungsquelle für die Brachytherapie in einer Applikatorspitze (8), wobei die Vorrichtung umfasst:
- eine Basisvorrichtung (1), die enthält:
- wenigstens zwei Strahlungsdetektoren (2),
- wenigstens zwei Kanäle (3), um die Strahlung zu führen,
- wenigstens zwei Öffnungen (4) in der Oberfläche der Basisvorrichtung (1), wobei jeder Kanal (3) einen Strahlungsdetektor (2) mit einer Öffnung (4) in der Oberfläche der Basisvorrichtung (1) verbindet, und
- einen Applikatorhalter (6), der einen Hohlraum (7) aufweist, um die Applikatorspitze (8) aufzunehmen, wobei der Hohlraum (7) ein distales Ende besitzt, das das distal äußerste Ende der Applikatorspitze (8), die die radioaktive Strahlungsquelle (9) enthält, aufnimmt, wobei der Applikatorhalter (6) ferner wenigstens zwei Öffnungen (4a, 4b) aufweist, die so angeordnet sind, dass sie mit den Öffnungen (4) in der Basisvorrichtung (1) übereinstimmen, und wenigstens zwei Kollimatorkanäle (5) aufweist, wobei die Kanäle (5) Verlängerungen der Kanäle (3) in der Basisvorrichtung (1) durch die Öffnungen (4) in der Basisvorrichtung (1) bilden und wobei die Kanäle (5) getrennte Punkte des Hohlraums (7) mit den Öffnungen in dem Applikatorhalter (6) verbinden, um Strahlung, die an getrennten Positionen des Hohlraums (7) zu dem jeweiligen Strahlungsdetektor (2) emittiert wird, zu führen und parallel zu richten, wobei sich einer der Punkte am distalen Ende des Hohlraums (7) befindet.

2. Testvorrichtung nach Anspruch 1, die ferner Kollimatoren umfasst, die in jedem Kanal (3, 5) wenigstens im Applikatorhalter angeordnet sind.

3. Testvorrichtung nach einem der vorhergehenden Ansprüche, bei der die Basisvorrichtung (1) ferner wenigstens eine Leistungsversorgung (11), einen Impulsformer, einen Impulszähler, Mittel zum Umsetzen der Ausgangsergebnisse der Strahlungsdetektoren in elektronische Signale, ein Ausgangsmittel für die detektierten Strahlungsmengen, eine Datenverarbeitungseinheit, eine Anzeige und eine Dateneingabeeinheit umfasst.

4. Testvorrichtung nach einem der vorhergehenden Ansprüche, bei der der Applikatorhalter (6) aus einem Strahlungsabschirmungsmaterial mit geeigneter Abschirmungskapazität für den Strahlungstyp, der von der zu testenden Quelle (9) emittiert wird, hergestellt ist.

5. Testvorrichtung nach einem der vorhergehenden Ansprüche, die zwei oder drei Strahlungsdetektoren (2) und zwei oder drei Kanäle (3), die jeden der Strahlungsdetektoren (2) mit getrennten Punkten des Hohlraums (7) in einer geraden Linie verbinden, umfasst.

6. Testvorrichtung nach Anspruch 5, wobei die Vorrichtung zwei Strahlungsdetektoren (2) und zwei Kanäle (3), die das distale Ende bzw. das proximale Ende des Hohlraums (7) mit jeweils einen der Strahlungsdetektoren (2) verbinden, umfasst.

7. Testvorrichtung nach Anspruch 5, wobei die Vorrichtung drei Strahlungsdetektoren (2) und drei Kanäle (3), die das distale Ende bzw. den mittleren Abschnitt bzw. das proximale Ende des Hohlraums (7) jeweils mit einem der Strahlungsdetektoren (2) verbinden, umfasst.

8. Testvorrichtung nach einem der vorhergehenden Ansprüche, bei der die Längsachse der Applikatorspitze (8) und jeder der Kanäle (3, 5) in derselben Ebene angeordnet sind.

9. Testvorrichtung nach einem der vorhergehenden Ansprüche, bei der der Applikatorhalter (6) an der Basisvorrichtung (1) in der Weise befestigt ist, dass die jeweiligen Öffnungen in der Basisvorrichtung (1) und in dem Applikatorhalter (6) aufeinander ausgerichtet sind.

10. Testvorrichtung nach einem der vorhergehenden Ansprüche, bei der der Applikatorhalter (6) aus zwei Teilen besteht, die optional durch ein Scharnier verbunden sind.

11. Testvorrichtung nach einem der vorhergehenden Ansprüche, bei der die Anzeige eine Balkendiagrammanzeige ist.

12. Verfahren zum Testen der richtigen Positionierung einer Strahlungsquelle für die Brachytherapie in einer Applikatorspitze (8), wobei das Verfahren die folgenden Schritte umfasst:
(i) Vorsehen einer Testvorrichtung nach einem der Ansprüche 1 bis 12, die umfasst:
- eine Basisvorrichtung (1), die enthält:
- wenigstens zwei Strahlungsdetektoren (2),
- wenigstens zwei Kanäle (3), um Strahlung zu führen,
- wenigstens zwei Öffnungen (4) in der Oberfläche der Basisvorrichtung (1), wobei jeder Kanal (3) einen Strahlungsdetektor (2) mit einer Öffnung (4) in der Oberfläche der Basisvorrichtung (1) verbindet, und
- einen Applikatorhalter (6), der einen Hohlraum (7) aufweist, um die Applikatorspitze (8) aufzunehmen, wobei der Hohlraum (7) ein distales Ende besitzt, das das distal äußerste Ende der Applikatorspitze (8), die die radioaktive Strahlungsquelle (9) enthält, aufnimmt, wobei der Applikatorhalter (6) ferner wenigstens zwei Öffnungen (4a, 4b) aufweist, die so angeordnet sind, dass sie mit den Öffnungen (4) in der Basisvorrichtung (1) übereinstimmen, und wenigstens zwei Kollimatorkanäle (5) aufweist, wobei die Kanäle (5) Verlängerungen der Kanäle (3) in der Basisvorrichtung (1) durch die Öffnungen (4) in der Basisvorrichtung (1) bilden und wobei die Kanäle (5) getrennte Punkte des Hohlraums (7) mit den Öffnungen in dem Applikatorhalter (6) verbinden, um Strahlung, die an getrennten Positionen des Hohlraums (7) zu dem jeweiligen Strahlungsdetektor (2) emittiert wird, zu führen und parallel zu richten, wobei sich einer der Punkte am distalen Ende des Hohlraums (7) befindet.
(ii) Einführen der Applikatorspitze (8), die die Strahlungsquelle (9) enthält, in den Applikatorhalter (6);
(iii) Befestigen des Applikatorhalters (6) an der Vorrichtung oder Ausrichten des Applikatorhalters (6) auf die Vorrichtung; und
(iv) Detektieren von emittierter Strahlung in den Strahlungsdetektoren (2).

13. Verfahren nach Anspruch 12, das ferner den Schritt des Vergleichens der detektierten Strahlung mit einer vorgegebenen Menge oder einem vorgegebenen Muster oder mit der Strahlung, die im Detektor, vorzugsweise in einem benachbarten weiteren Detektor, erfasst wird, umfasst.

14. Verfahren nach einem der Ansprüche 12 oder 13, das ferner den Schritt des Anzeigens der detektierten Strahlungsmenge und/oder des Vergleichsergebnisses umfasst.

15. Verfahren nach Anspruch 14, bei dem die Anzeige durch eine Balkendiagrammanzeige erfolgt, wobei die Länge des Balkens die an dieser Position emittierte Strahlungsmenge angibt.

16. Verfahren nach einem der Ansprüche 12 bis 15, das ferner den Schritt des Sterilisierens des Applikatorhalters (6) vor dem Einführen der Applikatorspitze (8) im Schritt (ii) umfasst.

17. Verfahren nach einem der Ansprüche 12 bis 16, bei dem die Basisvorrichtung (1) zwei Kanäle (3) und Strahlungsdetektoren (2) umfasst und die am distalen Ende und am proximalen Ende des Hohlraums (7) emittierte Strahlung detektiert wird.

18. Verfahren nach einem der Ansprüche 12 bis 16, bei dem die Basisvorrichtung (1) drei Kanäle (3) und Strahlungsdetektoren (2) umfasst und die am distalen Ende, im mittleren Teil und am proximalen Ende des Hohlraums (7) emittierte Strahlung detektiert wird.

19. Verfahren nach Anspruch 14, bei dem die Anzeige die Form eines Fehler/Bestanden-Signals hat.

## Revendications

1. Dispositif de test pour tester le positionnement correct d'une source de rayonnement radioactif pour brachythérapie dans une pointe d'applicateur (8), ledit dispositif comprenant
- un dispositif de base (1) contenant
- au moins deux détecteurs de rayonnement (2),
- au moins deux canaux (3) pour guider le rayonnement,
- au moins deux ouvertures (4) dans la surface du dispositif de base (1), chaque canal (3) connectant un détecteur de rayonnement (2) avec une ouverture (4) dans la surface du dispositif de base (1), et
- un support d'applicateur (6) comprenant une cavité (7) pour recevoir la pointe d'applicateur (8), la cavité (7) ayant une extrémité distale recevant l'extrémité la plus distale de la pointe d'applicateur (8) incluant la source de rayonnement radioactif (9), ledit support d'applicateur (6) incluant de plus au moins deux ouvertures (4a, 4b), qui sont agencées pour coïncider avec les ouvertures (4) dans le dispositif de base (1), au moins deux canaux de collimation (5), lesquels canaux (5) forment des extensions des canaux (3) dans le dispositif de base (1) à travers les ouvertures (4) dans le dispositif de base (1), et lesquels canaux (5) connectent des points séparés de la cavité (7) avec les ouvertures dans le support d'applicateur (6) pour guider et collimater le rayonnement émis dans des positions séparées de la cavité (7) au détecteur de rayonnement respectif (2), l'un de ces points est placé à l'extrémité distale de la cavité (7).

2. Dispositif de test de la revendication 1 comprenant de plus des collimateurs agencés dans chaque canal (3, 5) au moins dans le support d'applicateur.

3. Dispositif de test de l'une des revendications précédentes dans lequel le dispositif de base (1) comprend de plus au moins l'un d'une alimentation de puissance (11), d'un élément de formation d'impulsions, d'un compteur d'impulsions, d'un moyen pour convertir les résultats de sortie des détecteurs de rayonnement en signaux électroniques, d'un moyen de sortie pour les quantités de rayonnement détectées, d'une unité de traitement de données, d'un dispositif d'affichage, et d'une unité d'entrée de données.

4. Dispositif de test de l'une des revendications précédentes dans lequel le support d'applicateur (6) est fait en un matériau de protection contre le rayonnement d'une capacité de protection appropriée pour le type de rayonnement émis par la source (9) qui doit être testée.

5. Dispositif de test de l'une des revendications précédentes incluant deux ou trois détecteurs de rayonnement (2) et deux ou trois canaux (3), connectant en ligne droite chacun des détecteurs de rayonnement (2) avec des points séparés de la cavité (7).

6. Dispositif de test de la revendication 5 dans lequel le dispositif comprend deux détecteurs de rayonnement (2) et deux canaux (3) connectant l'extrémité distale et l'extrémité proximale de la cavité (7), respectivement, chacun avec l'un des détecteurs de rayonnement (2).

7. Dispositif de test de la revendication 5 dans lequel le dispositif comprend trois détecteurs de rayonnement (2) et trois canaux (3) connectant l'extrémité distale, la partie médiane et l'extrémité proximale de la cavité (7), respectivement, chacune avec l'un des détecteurs de rayonnement (2).

8. Dispositif de test de l'une des revendications précédentes dans lequel l'axe longitudinal de la pointe d'applicateur (8) et chacun des canaux (3, 5) sont agencés dans le même plan.

9. Dispositif de test de l'une des revendications précédentes dans lequel le support d'applicateur (6) est attaché au dispositif de base (1) de telle sorte que les ouvertures respectives dans le dispositif de base (1) et dans le support d'applicateur (6) soient alignées les unes par rapport aux autres.

10. Dispositif de test de l'une des revendications précédentes dans lequel le support d'applicateur (6) se compose de deux parties connectées optionnellement par une charnière.

11. Dispositif de test de l'une des revendications précédentes dans lequel le dispositif d'affichage est un dispositif d'affichage graphique à colonnes.

12. Procédé pour tester le positionnement correct d'une source de rayonnement pour brachythérapie dans une pointe d'applicateur (8), ledit procédé comprenant les étapes de :
(i) fournir un dispositif de test selon l'une des revendications 1 à 12 comprenant
- un dispositif de base (1) contenant
- au moins deux détecteurs de rayonnement (2),
- au moins deux canaux (3) pour guider le rayonnement,
- au moins deux ouvertures (4) dans la surface du dispositif de base (1), chaque canal (3) connectant un détecteur de rayonnement (2) avec une ouverture (4) dans la surface du dispositif de base (1), et
- un support d'applicateur (6) comprenant une cavité (7) pour recevoir la pointe d'applicateur (8), la cavité (7) ayant une extrémité distale recevant l'extrémité la plus distale de la pointe d'applicateur (8), incluant la source de rayonnement radioactif (9), ledit support d'applicateur (6) incluant de plus au moins deux ouvertures (4a, 4b), qui sont agencées pour coïncider avec les ouvertures (4) dans le dispositif de base (1), et au moins deux canaux (5), lesquels canaux (5) forment des extensions des canaux (3) dans le dispositif de base (1) à travers les ouvertures (4) dans le dispositif de base, et lesquels canaux (5) connectent des points séparés de la cavité (7) avec les ouvertures (4a, 4b) dans le support d'applicateur (6) pour guider et collimater le rayonnement émis dans des positions séparées de la cavité (7) au détecteur de rayonnement respectif (2), l'un de ces points est placé à l'extrémité distale de la cavité (7) ;
(ii) insérer la pointe d'applicateur (8) incluant la source de rayonnement (9) dans le support d'applicateur (6) ;
(iii) attacher le support d'applicateur (6) à ou l'aligner avec le dispositif ; et
(iv) détecter un rayonnement émis dans les détecteurs de rayonnement (2).

13. Procédé de la revendication 12 comprenant de plus l'étape consistant à comparer le rayonnement détecté avec une quantité ou modèle prédéterminé ou avec le rayonnement comme détecté dans le détecteur, de préférence un autre détecteur avoisinant.

14. Procédé de l'une des revendications 12 ou 13 comprenant de plus l'étape consistant à afficher la quantité détectée de rayonnement et/ou le résultat de la comparaison.

15. Procédé de la revendication 14 dans lequel l'affichage se produit par affichage graphique à colonnes, la longueur de la colonne indiquant la quantité de rayonnement émise à ladite position.

16. Procédé de l'une des revendications 12 à 15 comprenant de plus l'étape consistant à stériliser le support d'applicateur (6) avant d'insérer la pointe d'applicateur (8) de l'étape (ii).

17. Procédé de l'une des revendications 12 à 16 dans lequel le dispositif de base (1) comprend deux canaux (3) et détecteurs de rayonnement (2), et un rayonnement émis à l'extrémité distale et l'extrémité proximale de la cavité (7) est détecté.

18. Procédé de l'une des revendications 12 à 16 dans lequel le dispositif de base (1) comprend trois canaux (3) et détecteurs de rayonnement (2), et un rayonnement émis à l'extrémité distale, dans la partie médiane et à l'extrémité proximale de la cavité (7) est détecté.

19. Procédé de la revendication 14, dans lequel l'affichage est en forme d'un signal d'échec/succès.
